# FASCICULE DE BREVET EUROPEEN

(11) **EP 0 943 323 B1**
(45) Date de publication et mention de la délivrance du brevet: **19.11.2003**
(21) Numéro de dépôt: 99400335.8
(22) Date de dépôt: 11.02.1999
(51) Int. Cl.: A61K 7/48, A61K 7/06, A61K 35/56, A61K 38/17, C07K 14/435

(54) **Composition cosmétique ou dermatologique contenant au moins une protéine de soie d'arachnides naturelle, recombinante ou un analogue**
Kosmetische oder dermatologische Zusammensetzung, enthaltend zumindest ein natürliches oder rekombinantes Seidenprotein von Arachniden oder ein analoges Protein
Cosmetic or dermatological composition containing at least a natural or recombinant arachnida silk protein or an analogue

(30) Priorité: 11.02.1998 FR 9801614
(43) Date de publication de la demande: 22.09.1999
(73) Titulaire: L'OREAL, 75008 Paris (FR)
(72) Inventeur: Philippe, Michel, 91320 Wissous (FR); Garson, Jean-Claude, 92150 Suresnes (FR); Arraudeau, Jean-Pierre, 75015 Paris (FR)
(74) Mandataire: Tezier Herman, Béatrice

(56) Documents cités:
- EP-A- 0 452 925
- WO-A-91/16351
- WO-A-97/08315

## Description

La présente invention a pour objet une nouvelle composition, cosmétique ou dermatologique, contenant en tant que substance active au moins une protéine de soie d'arachnides, naturelle ou recombinante, ou un analogue d'une telle protéine.

Plus particulièrement, la présente invention se rapporte à des compositions sous forme de produits capillaires, de produits de soins pour la peau, de produits de maquillage, ou de produits pour la protection solaire à base d'une protéine de soie d'arachnides.

L'utilisation de protéines naturelles ou végétales est bien connue et a été largement pratiquée dans le domaine cosmétique, notamment, l'utilisation de protéines de soie de l'insecte Bombyx mori, plus particulièrement décrite dans Biomaterials Ed. D. Byrom, Stocktompress, 1-54, (1991). Toutefois, ces protéines de soie ne présentent pas de bonnes propriétés mécaniques à l'état de fils ou de films et ne peuvent donc remplir de manière satisfaisante leurs fonctions d'agents filmogènes.

Les protéines de soie d'arachnides ont été caractérisées par Lucas et al., Adv. Protein Chem. 13:107-242, (1958) ; Andersen, Comp. Biochem. Physiol. 35:705-711, (1970) ; Denny MW, J. Exp. Biol., 65, 483-506, (1976) ; et Lucas F, Discovery, 25, 20-26, (1964). Elles sont connues pour leurs bonnes propriétés mécaniques et élastiques.

On a maintenant découvert qu'en plus de leurs propriétés connues, les protéines de soie d'arachnides conféraient aux compositions cosmétiques ou dermatologiques des propriétés inattendues notamment une amélioration de l'effet hydratant ou plastifiant. Les protéines de soie d'arachnides se comportent par ailleurs comme de bons agents filmogènes rémanents à l'eau, et surtout présentent une faible densité de surface.

La présente invention a donc pour objet à titre de produit industriel nouveau, une composition cosmétique ou dermatologique contenant une quantité efficace d'au moins une protéine de soie d'arachnides, naturelle ou recombinante ou d'un analogue d'une telle protéine.

Selon une forme particulière de réalisation des compositions cosmétiques selon la présente invention, les protéines de soie d'arachnides sont de préférence des protéines de soie d'arachnides recombinantes.

Bien que les protéines naturelles puissent être utilisées selon l'invention pour la formulation de produits cosmétiques ou dermatologiques notamment du fait de leur bonne biocompatibilité et biodégradabilité, les gènes codant ces protéines de soie présentent une certaine instabilité génétique due aux répétitions des séquences nucléotidiques ne permettant pas d'obtenir une bonne reproductibilité de leurs propriétés physicochimiques.

Par contre, les protéines de soie d'arachnides recombinantes ont l'avantage de présenter une séquence peptidique prédéterminée que l'on peut concevoir en fonction des propriétés physico-chimiques de résistance, ou encore d'élasticité requises pour les diverses applications cosmétiques ou dermatologiques souhaitées.

Selon l'invention, les compositions peuvent contenir en une quantité efficace au moins une protéine de soie d'arachnides, naturelle ou recombinante, correspondant à la Spidroine majeure 1 décrite par Xu et al., PNAS, USA, 87, 7120, (1990), à la Spidroine majeure 2 décrite par Hinman et Lewis, J. Biol. Chem., 267, 19320, (1992), ou encore aux Spidroines mineures décrites dans la demande WO 95/25165. Les séquences peptidiques sont fournies dans les SEQ ID N°1, 2 et 3 de la présente demande qui illustrent respectivement la séquence peptidique d'une protéine de soie d'arachnides naturelle, Spidroine majeure 1, la séquence peptidique d'une protéine de soie d'arachnides naturelle, Spidroine majeure 2 et la séquence peptidique correspondant aux résidus 92 à 706 de la protéine de soie d'arachnides naturelle, Spidroine mineure 1.

Lorsque les compositions selon la présente invention contiennent au moins une protéine de soie d'arachnides recombinante, celle-ci comprend une unité répétitive d'acides aminés répondant à l'une des séquences (I), (II), (III) et/ou (IV) ci dessous.

Les abréviations suivantes sont utilisées pour identifier les acides aminés :

| **Acides aminés** | **Abréviations** |
|---|---|
| Alanine | A |
| Glycine | G |
| Proline | P |
| Sérine | S |
| Glutamine | Q |
| Tyrosine | Y |
| Arginine | R |
| Asparagine | N |
| Acide aspartique | D |
| Cystéine | C |
| Acide glutamique | E |
| Histidine | H |
| Leucine | L |
| Lysine | K |
| Méthionine | M |
| Phénylalanine | F |
| Thréonine | T |
| Tryptophane | W |
| Valine | V |
| Isoleucine | I |

[(XGG)_{w}(XGA)(GXG)ₓ(AGA)_{y}(G)_{z}AG]ₚ (I)

dans laquelle :
X correspond à la Tyrosine ou à la Glutamine,
w est un nombre entier égal à 2 ou 3,
x est un nombre entier de 1 à 3,
y est un nombre entier de 5 à 7,
z est un nombre entier égal à 1 ou 2, et
p est un nombre entier tel que le poids moléculaire de la protéine soit compris entre 10 et 400 Kda,
et/ou

[(GPG₂YGPGQ₂)ₐ(X')₂S(A)_{b}]ₚ (II)

dans laquelle :
X' correspond à la séquence d'acides aminés GPS ou GPG,
a est égal à 2 ou 3,
b est un nombre entier de 7 à 10, et
p est un nombre entier tel que le poids moléculaire de la protéine soit compris entre 10 et 400 Kda,
et/ou

[(GR)(GA)₁(A)ₘ(GGX")ₙ(GA)ₗ(A)ₘ]ₚ (III)

et/ou

[(GGX")ₙ(GA)ₘ(A)ₗ]ₚ (IV)

dans lesquelles :
X" correspond à la Tyrosine, la Glutamine ou l'Alanine,
l est un nombre entier de 1 à 6,
m est un nombre entier de 0 à 4,
n est un nombre entier de 1 à 4, et
p est un nombre entier tel que le poids moléculaire de la protéine soit compris entre 10 et 400 Kda.

Selon une autre forme de réalisation de la présente invention, les compositions cosmétiques ou dermatologiques peuvent contenir une quantité efficace d'au moins un analogue d'une protéine de soie d'arachnides.

Par "analogue", il faut entendre selon l'invention, des peptides qui imitent les unités répétitives d'acides aminés dérivées des protéines de soie d'arachnides naturelles et le profil de variation entre les unités répétitives sans modifier leur conformation tridimensionnelle. Il est ainsi possible de concevoir une séquence peptidique de façon à obtenir les propriétés physico-chimiques requises pour les diverses applications cosmétiques ou dermatologiques souhaitées.

A ce sujet, les demandes WO 91/16351 et EP 452 925 décrivent la synthèse de peptides analogues présentant des propriétés d'élasticité supérieures du fait de l'allongement de la séquence de polyalanine. A l'inverse, il est possible de réduire l'élasticité de la protéine par l'élimination des séquences de polyalanine. De même, lorsque l'on désire obtenir une protéine moins rigide, il suffit de substituer les résidus de glycine par des résidus de sérine.

Par ailleurs, la demande WO 94/29450 décrit la synthèse de peptides analogues contenant les codons préférentiels et spécifiques de l'organisme hôte. En effet, il est bien connu que l'expression de gènes étrangers est plus efficace lorsque le codon à usage préférentiel de l'organisme hôte est utilisé. De tels peptides analogues peuvent être synthétisés à partir de monomères d'ADN de 300 à 400 bp codant pour une ou plusieurs unités répétitives d'acides aminés, qui sont ensuite associés pour former un gène complet codant pour un analogue. Des exemples de séquences de peptides analogues sont fournis dans les SEQ ID N° 4, 5, 6, 7, 8, 9, 10 et 11 de la présente demande. Les SEQ ID N° 4 et 5 illustrent respectivement la séquence d'acides aminés d'un monomère et d'un polymère d'un analogue de la Spidroine majeure 1. Les SEQ ID N° 6 et 7 illustrent respectivement la séquence d'acides aminés d'un monomère et d'un polymère d'un analogue de la Spidroine majeure 1. Les SEQ ID N° 8 et 9 illustrent respectivement la séquence d'acides aminés d'un monomère et d'un polymère d'un analogue de la Spidroine majeure 1. Les SEQ ID N° 10 et 11 illustrent respectivement la séquence d'acides aminés d'un monomère et d'un polymère d'un analogue de la Spidroine majeure 2.

Les différentes méthodes de synthèse de ces peptides analogues sont connues et ont été décrites par Ausubel et al., Current Protocols in Molecular Biology § 8 (John Wiley & Sons 1987, (1990)). Il s'agit, entre autres, de la mutagenèse dirigée de polynucléotides codant pour un fragment fonctionnel d'une protéine de soie d'arachnides.

Les protéines de soie d'arachnides naturelles peuvent être recueillies selon la méthode décrite par Work et al., J. Arachrol., 10,1-10, (1982) et dans la demande EP 452 925.

Les protéines recombinantes peuvent être produites par des systèmes procaryotes ou eucaryotes transformés contenant le cDNA codant pour une protéine de soie d'arachnides, pour un frag.ment de cette protéine, ou encore pour un analogue d'une telle protéine.

Les systèmes d'expression des protéines sont bien connus et ont été décrits par Maniatis Molecular Cloning, A Lab Manual (1982) et Sambrook et al., Molecular Cloning, volumes 1, 2, 3 (Cold Spring Harbor Laboratory: Cold Spring Harbor, New York, (1989)).

Les systèmes procaryotes incluent les bactéries gram négatif ou les bactéries gram positif, telles que *Escherichia coli* ou *Bacillus subtilis*. Les vecteurs d'expression procaryotiques peuvent comporter une origine de réplication reconnaissable par l'organisme hôte, un promoteur homologue ou hétérologue et fonctionnel dans ledit hôte, la séquence d'ADN codant pour la protéine de soie d'arachnides, pour un fragment de cette protéine, ou pour une protéine analogue, et un gène de sélection tel qu'un gène de résistance antibiotique.

Les systèmes eucaryotes incluent les levures (*Saccharomyces cerevisiae*), les cellules d'insectes, de mammifères ou de plantes. Les vecteurs d'expression peuvent alors comporter une origine de réplication du plasmide de la levure ou une séquence de réplication autonome, un promoteur, une séquence d'ADN codant pour une protéine de soie d'arachnides, pour un fragment, ou pour une protéine analogue, une séquence de polyadénylation, un site de terminaison de transcription et enfin un gène de sélection.

Dans les compositions de l'invention, l'expression "quantité efficace" d'au moins une protéine de soie d'arachnides naturelle ou recombinante, ou d'un analogue, correspond à une proportion d'environ 10⁻⁴ à 30 % en poids, mais de préférence de 10⁻³ à 15 % en poids par rapport au poids total de la composition, cette proportion pouvant varier en fonction du type de composition cosmétique ou dermatologique.

Selon un premier mode de réalisation particulier des compositions selon l'invention, celles-ci sont anhydres et comprennent une phase grasse en une proportion généralement d'environ 10 à 90 % en poids par rapport au poids total de la composition, ladite phase grasse contenant au moins un corps gras liquide, solide ou semi-solide.

Les corps gras peuvent être choisis parmi les huiles, les cires, les gommes et/ou les corps gras dits pâteux.
**A** - Les huiles de la phase grasse peuvent être d'origine minérale, animale, végétale ou de synthèse, celles-ci pouvant être volatiles ou non à température ambiante.
   Comme huile d'origine minérale, on peut citer notamment l'huile de paraffine et l'huile de vaseline.
   Comme huile d'origine animale, on peut citer notamment le squalène et le squalane.
   Comme huile d'origine végétale, on peut citer notamment l'huile d'amande douce, l'huile de calophyllum, l'huile de palme, l'huile d'avocat, l'huile de jojoba, l'huile de sésame, l'huile d'olive, l'huile de ricin et les huiles de germes de céréales telles que par exemple l'huile de germes de blé.
   Comme huile de synthèse, on peut citer notamment :
   (1) les esters de formule suivante :

      R₁-COOR₂

      dans laquelle :
      R₁ représente le reste d'un acide gras supérieur ayant de 7 à 20 atomes de carbone et
      R₂ représente un radical hydrocarboné ayant de 3 à 30 atomes de carbone.

      Parmi ces esters, on peut notamment citer : l'huile de Purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, l'isononanoate d'isononyle, les esters dérivés de l'acide lanolique tels que le lanolate d'isopropyle et le lanolate d'isocétyle.
      Comme autres huiles de synthèse, on peut citer en outre l'isododécane, l'isohexadécane, les polyisobutènes et le polyisobutène hydrogéné ainsi que les acétylglycérides, les octanoates et décanoates de polyalcools tels que ceux de glycol et de glycérol, les ricinoléates d'alcools ou de polyalcools tels que le ricinoléate de cétyle, le dicaprylate de propylène glycol et l'adipate de diisopropyle ;
   (2) les alcools gras tels que l'alcool oléique, l'alcool linoléique, l'alcool linolénique, l'alcool isostéarylique et l'octyldodécanol ;
   (3) les huiles de silicone telles que les polydiorganosiloxanes linéaires éventuellement fonctionnalisés, les polydiorganosiloxanes cycliques et en particulier les cyclotétra- et penta-diméthicones et les organopolysiloxanes tels que les alkyl, alcoxy ou phényl diméthicones et en particulier la phényltriméthicone ;
   (4) les huiles fluorées telles que les perfluoroalcanes et les perfluoropolyéthers et les huiles hydrocarbonées partiellement fluorées.
      **B** - Les cires de la phase grasse peuvent être d'origine minérale, fossile, animale, végétale, de synthèse ou bien encore être des huiles hydrogénées ou des esters gras concrets à 25°C.

   Parmi les cires minérales, on peut citer notamment les cires microcristallines, la paraffine, la vaseline et la cérésine.
   Parmi les cires fossiles, on peut citer l'ozokérite et la cire de montan.
   Parmi les cires d'origine animale, on peut citer la cire d'abeilles, le spermaceti, la cire de lanoline ainsi que les dérivés issus de la lanoline tels que les alcools de lanoline, la lanoline hydrogénée, la lanoline hydroxylée, la lanoline acétylée, les acides gras de lanoline et l'alcool de lanoline acétylé.
   Parmi les cires d'origine végétale, on peut citer notamment la cire de candellila, la cire de carnauba, la cire du Japon et le beurre de cacao.
   Parmi les cires de synthèse, on peut citer notamment les homopolymères d'éthylène et les copolymères d'éthylène et d'un monomère répondant à la formule :

   CH₂=CH-R₃

   dans laquelle :
   R₃ représente un radical alkyle ayant de 1 à 30 atomes de carbone, un radical aryle ou aralkyle.
   Le radical alkyle de 1 à 30 atomes de carbone est de préférence le radical méthyle, éthyle, propyle, isopropyle, butyle, décyle, dodécyle ou octadécyle.
   On peut également utiliser des cires obtenues par synthèse Fisher-Tropsch ainsi que des cires de silicone.

   Parmi les huiles hydrogénées concrètes à 25°C, on peut citer notamment l'huile de ricin hydrogénée, l'huile de palme hydrogénée, le suif hydrogéné et l'huile de coco hydrogénée.
   Parmi les esters gras concrets à 25°C, on peut citer notamment le mono-myristate de propylène glycol et le myristate de myristyle.
   Comme cires utilisables dans les compositions selon l'invention, on peut en outre citer l'alcool cétylique, l'alcool stéarylique, les mono-, di- et triglycérides concrets à 25°C, le monoéthanolamide stéarique, la colophane et ses dérivés tels que les abiétates de glycol et de glycérol, les sucro-glycérides et les oléates, myristates, lanolates, stéarates et dihydroxystéarates de calcium, de magnésium, de zinc et d'aluminium.
**C** - Les corps gras de type pâteux peuvent être d'origine minérale, animale, végétale ou de synthèse.

Parmi les corps gras pâteux, on peut citer notamment les esters de synthèse tels que le propionate d'arachidyle, le polylaurate de vinyle, les cires de polyéthylène et les organopolysiloxanes tels que les alkyldiméthicones, les alcoxydiméthicones ou les esters de diméthicones.

Les compositions anhydres selon l'invention peuvent bien entendu contenir en outre un ou plusieurs additifs ou adjuvants cosmétiques ou dermatologiques conventionnels.

Ces compositions anhydres peuvent se présenter sous différentes formes telles que notamment d'un gel huileux, de produits solides tels que des poudres compactées ou coulées, ou bien encore de sticks tels que par exemple des rouges à lèvres.

Lorsque les compositions selon l'invention se présentent sous forme d'un gel huileux, elles contiennent généralement, outre les constituants définis précédemment, un gélifiant huileux.

Parmi les gélifiants huileux, on peut notamment citer les esters métalliques tels que le stéarate de polyoxyaluminium et l'hydroxystéarate d'aluminium ou de magnésium, les esters d'acide gras et de glycol, les triglycérides, les mélanges d'alcools gras, les dérivés de cholestérol et en particulier l'hydroxycholestérol, et les minéraux argileux gonflants en présence d'huile et en particulier ceux appartenant au groupe des montmorillonites.

Les gélifiants huileux peuvent être présents en une proportion très variable selon la texture des compositions recherchée. Toutefois, dans la plupart des cas, ils sont présents en une proportion d'environ 0,1 à 30 % en poids par rapport au poids total de la composition.

Ces compositions anhydres selon l'invention peuvent être utilisées notamment comme produits de soins, de nettoyage, ou de maquillage.

Lorsqu'elles se présentent sous forme de produits de maquillage, elles peuvent être en particulier des fonds de teint, des mascaras, des eyeliners, des rouges à lèvres, des fards à paupières ou à joues. Ces compositions sont généralement colorées et contiennent alors en tant qu'adjuvants cosmétiques, des colorants et/ou des pigments bien connus dans le domaine des produits de maquillage.

Selon un deuxième mode de réalisation des compositions selon l'invention, celles-ci sont des dispersions sous forme d'une émulsion eau-dans-l'huile (E/H) ou huile-dans-l'eau (H/E), stables, qui sont essentiellement constituées (i) d'une phase grasse en une proportion d'environ 0,1 à 50 % en poids par rapport au poids total de la composition, ladite phase grasse contenant au moins une protéine de soie d'arachnides naturelle ou recombinante, ou un analogue en une proportion d'environ 10⁻⁴ à 30 % en poids par rapport au poids total de l'émulsion, (ii) d'une phase aqueuse en une proportion d'environ 50 à 98,9 % en poids par rapport au poids total de l'émulsion, et (iii) d'au moins un agent émulsionnant en une proportion d'environ 1 à 10 % en poids par rapport au poids total de l'émulsion.

Ces émulsions qui se présentent sous forme de crèmes possèdent un bon caractère filmogène et laissent une sensation de confort très satisfaisant après application. De telles émulsions peuvent être utilisées comme produits de soins, de nettoyage ou de maquillage.

Lorsque ces compositions sont des produits de soins pour la peau, celles-ci peuvent être notamment des produits antirides permettant d'améliorer l'aspect du relief cutané.

Lorsque ces compositions sont des produits de maquillage, celles-ci peuvent être notamment des fonds de teint ou des mascaras contenant alors en une certaine proportion des pigments et/ou des colorants.

Selon un troisième mode de réalisation des compositions selon l'invention, celles-ci se présentent sous forme de produits pour les ongles tels que des vernis à ongles ou des produits de soin pour les ongles.

Selon ce mode de réalisation, les compositions se présentent de préférence sous forme de vernis à ongles contenant :
(i) un système solvant pour vernis, et
(ii)une substance filmogène.

La protéine de soie d'arachnides, naturelle ou recombinante ou analogue est selon cette forme de réalisation, généralement présente en une proportion de 10⁻⁴ à 30 % en poids par rapport au poids total du vernis.

Selon ce mode de réalisation, le système solvant du vernis est généralement présent en une proportion allant de 55 à 90 % en poids par rapport au poids total du vernis.

Bien que le système solvant puisse être du type aqueux, celui-ci est de préférence constitué par un mélange de divers solvants organiques volatils, ceci en vue d'obtenir des temps de séchage relativement courts.

Parmi ces solvants, on peut citer l'acétone, l'acétate d'éthyle, l'acétate de butyle, l'acétate de méthoxy-2 éthyle, la méthyl-éthyl-cétone, la méthyl-isobutyl-cétone, l'acétate de méthyle, l'acétate d'amyle et l'acétate d'isopropyle.

Le système solvant peut comprendre également un diluant qui est de préférence un hydrocarbure, linéaire ou ramifié, saturé, tel que l'hexane ou l'octane, ou encore un hydrocarbure aromatique tel que le toluène ou le xylène dans une proportion d'environ 10 à 35 % en poids par rapport au poids total du vernis. Le système solvant peut également inclure d'autres solvants volatils tels que l'éthanol, le n-butanol, le n-propanol, l'isopropanol ou leurs mélanges.

Outre la protéine de soie d'arachnides, la composition selon l'invention peut également comprendre une substance filmogène. Cette substance filmogène est généralement présente en une proportion d'environ 5 à 35 % en poids par rapport au poids total du vernis. Parmi ces substances filmogènes, on peut citer les nitrocelluloses du type "RS" ou "SS" et en particulier la nitrocellulose type ¼ seconde RS, la nitrocellulose type ¼ seconde RS, la nitrocellulose type ½ seconde SS et la nitrocellulose type ¾ seconde RS. Comme substance filmogène, on peut également utiliser selon l'invention des dérivés polyvinyliques tels que le butyrate de polyvinyle.

Comme autre substance filmogène, on peut utiliser selon l'invention des dérivés cellulosiques différents de la nitrocellulose, des polymères ou copolymères acryliques, des résines du type acrylique, styrénique, acrylate-styrénique et vinylique, des copolymères vinyliques, des polymères polyesters, des résines du type aryl-sulfonamide et des résines alkydes.

Les vernis selon l'invention peuvent également contenir un agent plastifiant qui est généralement présent en une proportion d'environ 5 à 20 % en poids par rapport au poids total du vernis. Les agents plastifiants permettent de régler la flexibilité du film sans affaiblir sa résistance ou sa force physique. Parmi les agents plastifiants, on peut citer : le phosphate de tricrésyle, le benzoate de benzyle, le phosphate de tributyle, l'acétyl ricinoléate de butyle, le citrate de triéthyle, l'acétyl citrate de tributyle, le phtalate de dibutyle et le camphre.

Les produits pour les ongles selon l'invention peuvent être soit incolores soit colorés. Lorsqu'ils sont colorés, ils contiennent alors des pigments et/ou des colorants bien connus dans le domaine des vernis à ongles.

Selon un dernier mode de réalisation particulier des compositions cosmétiques selon l'invention, celles-ci sont des compositions capillaires contenant dans un véhicule cosmétique approprié, une protéine de soie d'arachnides naturelle ou recombinante ou un analogue, en une proportion d'environ 10⁻⁴ à 30 % en poids par rapport au poids total de la composition.

Ces compositions capillaires peuvent être des solutions aqueuses, alcooliques ou hydroalcooliques, l'alcool étant de préférence l'éthanol ou l'isopropanol en une proportion d'environ 60 à 99,5 % en poids par rapport au poids total, pour les solutions hydroalcooliques.

Ces compositions capillaires peuvent se présenter sous forme de lotions, d'aérosols, de gels, de mousses ou de shampooings fixants. Elles s'appliquent facilement sur la chevelure et forment un film hydrophobe de faible densité à la surface des cheveux, permettant une mise en forme satisfaisante de la coiffure sans effet de poissage.

Les compositions selon l'invention, telles qu'elles viennent d'être décrites ci-dessus, du fait d'un fin dépôt d'un film hydrophobe sur la peau, les cheveux ou les ongles, permettent la vectorisation de divers actifs d'application .généralement difficile, tels que des vitamines, des hormones, des agents hydratants, et des produits actifs contre les désordres cutanés ou capillaires.

Les compositions selon l'invention contiennent également un ou plusieurs adjuvants cosmétiques conventionnels tels que des agents antioxydants, des conservateurs, des charges, des filtres solaires UVA et/ou UVB, des parfums, des épaississants, des agents humectants, des polymères anioniques, non-ioniques ou amphotères.

Parmi ces adjuvants, les charges sont généralement présentes, dans les produits de maquillage ou de soins ou dans les produits dermatologiques, en une proportion maximum d'environ 99,9 % en poids par rapport au poids total de la composition.

Ces charges, sous forme de poudres très fines, peuvent être d'origine naturelle ou synthétique. Parmi celles-ci, on peut notamment citer :
a) les poudres minérales telles que le talc, le kaolin, le mica, la silice, les silicates, l'alumine, les zéolites, l'hydroxyapatite, la séricite, le dioxyde de titane, les micatitanes, le sulfate de baryum, l'oxychlorure de bismuth, le nitrure de bore et les poudres métalliques telles que la poudre d'aluminium ;
b) les poudres végétales telles que les poudres d'amidon de maïs, de froment ou de riz ;
c) les poudres organiques telles que les poudres'de nylon, de polyamide, de polyester, de polytétrafluoroéthylène ou de polyéthylène.

Ces différentes poudres peuvent en outre être enrobées par exemple par des sels métalliques d'acides gras, des acides aminés, de la lécithine, du collagène, des composés siliconés, des composés fluorés, ou par tout autre enrobage usuel.

Outre les charges, les colorants et pigments entrent également dans les compositions de maquillage anhydres ou sous forme de dispersions. Les compositions de l'invention permettent, par enrobage, d'améliorer les propriétés de résistance à l'eau de ces composés fragiles. Les colorants et/ou pigments sont généralement présents en une proportion maximum d'environ 40 % par rapport au poids total de la composition.

Dans les rouges à lèvres, la proportion en au moins un colorant et/ou pigment est généralement d'environ 0,1 à 15 % en poids par rapport au poids total du rouge à lèvres.

Parmi les colorants des produits de maquillage et notamment des rouges à lèvres, on peut citer les dérivés d'éosine tels que le D&C Red n° 21 et les dérivés de fluorescéine halogénés tels que le D&C Red n° 27, le D&C Red Orange n° 5 en association avec le D&C Red n° 21 et le D&C Orange n° 10.

Parmi les pigments qui peuvent être minéraux ou organiques ou encore des laques métalliques, on peut citer le dioxyde de titane, l'oxyde de zinc, le D&C Red n° 36 et le D&C Orange n° 17, les laques de calcium de D&C Red n° 7, 11, 31 et 34, la laque de baryum de D&C Red n° 12, la laque de strontium D&C Red n° 13, les laques d'aluminium de FD&C Yellow n° 5, de FD&C Yellow n° 6, de D&C Red n° 27, de D&C Red n° 21, de FD&C Blue n° 1, les oxydes de fer, le violet de manganèse, l'oxyde de chrome et le bleu d'outre-mer.

Parmi les pigments des vernis à ongles les plus employés, on peut citer les D&C Red n° 8, 10, 30 et 36, les laques de baryum des D&C Red n° 6, 9 et 12, les laques de calcium des D&C Red n° 7, 11, 31 et 34, la laque de strontium du D&C Red n° 30 ainsi que le D&C Orange n° 17 et le D&C Blue n° 6, ces pigments étant généralement présents en une proportion comprise entre 0,01 et 2 % par rapport au poids total de la composition de vernis.

Les vernis à ongles peuvent également contenir du dioxyde de titane en vue d'impartir aux vernis une certaine opacité ainsi que certaines substances iridescentes telles que la guanine et des agents thixotropes permettant d'éviter la sédimentation des pigments tels que des argiles de montmorillonite modifiées comme par exemple la Bentone 27, la Bentone 34 ou la Bentone 38.

L'invention sera maintenant illustrée par les différents exemples suivants dont les quantités sont exprimées en poids.

### EXEMPLES DE COMPOSITION

### EXEMPLE 1 : Shampooing

On prépare un shampooing en procédant au mélange des ingrédients suivants :
- Lauryl sulfate d'ammonium 12 g (MA)
- Chlorure de sodium 1 g
- Acide polyacrylique réticulé vendu sous la dénomination de "CARBOPOL 980®" par la Société GOODRICH 3 g (MA)
- Hydroxyéthyl cellulose réticulé à l'épichlorhydrine quaternisé par triméthylamine vendue sous la dénomination de "CELQUAT SC 40®" par la Société NATIONAL STARCH 1 g (MA)
- Protéine de soie d'arachnides 3 g
- NaOH, q.s.p. pH 5
- Conservateurs, parfum, q.s.p.
- Eau, q.s.p 100 g
MA = matière active

### EXEMPLE 2 : Composition d'après-shampooing

On prépare une composition d'après-shampooing en procédant au mélange des ingrédients suivants :
- Copolymère chlorure de méthacrylate de triméthyléthyl ammonium/acrylamide (42/58) réticulé en dispersion à 50 % dans l'huile vendu sous la dénomination de "SALCARE SC92®" par la Société ALLIED COLLOIDS. 2 g (MA)
- Mélange alcool cétylique/stéarylique oxyéthyléné à 30 moles d'oxyde d'éthylène 2 g (MA)
- Protéine de soie d'arachnides 5 g
- HCl, q.s.p. pH 6
- Eau, q.s.p. 100 g

### EXEMPLE 3 : Lotion de soin pour les cheveux non rincée

On prépare une composition de soin pour les cheveux en procédant au mélange des ingrédients suivants :
- Copolymère acide acrylique/acrylate d'alkyle C10/C30 vendu sous la dénomination de "CARBOPOL 1392®" par la Société GOODRICH 2 g (MA)
- Protéine de soie d'arachnides 2 g
- Alcool éthylique 5 g
- NaOH, q.s.p. pH 6
- Eau, q.s.p. 100 g

### EXEMPLE 4 : Vernis à ongles

On prépare un vernis à ongles en procédant au mélange des ingrédients suivants :
- Nitrocellulose 16 g
- Acétyltributyl citrate 6 g
- Résine toluène sulfonamide formaldéhyde 10 g
- Argile bentonite 1,5 g
- Pigments 1,5 g
- Alcool isopropylique 3 g
- Protéine de soie d'arachnides recombinante 0 , 5 g
- Solvants (acétate d'éthyle et acétate de butyle) q.s.p. 100 g

### EXEMPLE 5 : Fond de teint (émulsion eau-dans-l'huile)

On prépare un fond de teint en procédant au mélange des ingrédients suivants :
- Copolymère de cétyl diméthicone et diméthicone copolyol vendu sous la dénomination de "ABIL WE09®" par la Société GOLDSCHMIDT 5 g
- Cyclométhicone 15 g
- Triglycéride caprylique/caprique vendu sous la dénomination de "MIGLYOL 812®" par la Société HÜLS AG 5 g
- Bentone gel VS 38 (argile à 10 % dans cyclométhicone) 10 g
- Protéine de soie d'arachnides recombinante 1 g
- Conservateurs, q.s.p.
- Pigments 7 g
- Eau, q.s.p 100 g

On réalise le mélange à froid à l'aide d'une turbine et la protéine de soie est incorporée après émulsification en agitant pendant 5 minutes.

## Revendications

1. Composition cosmétique ou dermatologique **caractérisée par le fait qu'**elle contient une quantité efficace d'au moins une protéine de soie d'arachnides naturelle ou recombinante ou un analogue d'une telle protéine et au moins un adjuvant cosmétique conventionnel choisi dans le groupe constitué par les charges, les filtres solaires UVA et/ou UVB, les agents thixotropes, les agents antioxydants, les conservateurs, les colorants, les pigments, les parfums, les épaississants, les vitamines, les hormones, les agents hydratants, les agents humectants, les polymères anioniques, non-ioniques ou amphotères et les substances actives dermatologiques.

2. Composition cosmétique ou dermatologique selon la revendication 1, **caractérisée par le fait que** ladite protéine de soie d'arachnides comprend une unité répétitive d'acides aminés de séquence (I) suivante :
[(XGG)_{w}(XGA)(GXG)ₓ(AGA)_{y}(G)_{z}AG]ₚ (I)
dans laquelle :
X correspond à la Tyrosine ou à la Glutamine,
G correspond à la Glycine,
A correspond à l'Alanine,
w est un nombre entier égal à 2 ou 3,
x est un nombre entier de 1 à 3,
y est un nombre entier de 5 à 7,
z est un nombre entier égal à 1 ou 2, et
p est un nombre entier tel que le poids moléculaire de la protéine soit compris entre 10 et 400 Kda.

3. Composition cosmétique ou dermatologique selon la revendication 2, **caractérisée par le fait que** ladite protéine de soie d'arachnides répond à la séquence peptidique SEQ ID N° 1.

4. Composition cosmétique ou dermatologique selon la revendication 1, **caractérisée par le fait que** ladite protéine de soie d'arachnides comprend une unité répétitive d'acides aminés de séquence (II) suivante:
[(GPG₂YGPGQ₂)ₐ(X')₂S(A)_{b}]ₚ (II)
dans laquelle :
X' correspond à la séquence d'acides aminés GPS ou GPG,
G correspond à la Glycine,
A correspond à l'Alanine,
P correspond à la Proline,
Y correspond à la Tyrosine,
Q correspond à la Glutamine,
S correspond à la Sérine,
a est égal à 2 ou 3,
b est un nombre entier de 7 à 10, et
p est un nombre entier tel que le poids moléculaire de la protéine soit compris entre 10 et 400 Kda.

5. Composition cosmétique ou dermatologique selon la revendication 4, **caractérisée par le fait que** ladite protéine de soie d'arachnides répond à la séquence polypeptidique SEQ ID N° 2.

6. Composition cosmétique ou dermatologique selon la revendication 1, **caractérisée par le fait que** ladite protéine de soie d'arachnides comprend une unité répétitive d'acides aminés de séquences (III) et/ou (IV) suivantes:
[(GR)(GA)₁(A)ₘ(GGX")ₙ(GA)ₗ(A)ₘ]ₚ (III)
et/ou
[(GGX")ₙ(GA)ₘ(A)ₗ]ₚ (IV)
dans lesquelles :
X" correspond à la Tyrosine, la Glutamine ou l'Alanine,
G correspond à la Glycine,
A correspond à l'Alanine,
R correspond à l'Arginine,
1 est un nombre entier de 1 à 6,
m est un nombre entier de 0 à 4,
n est un nombre entier allant de 1 à 4, et
p est un nombre entier tel que le poids moléculaire de la protéine soit compris entre 10 et 400 Kda.

7. Composition cosmétique ou dermatologique selon la revendication 6, **caractérisée par le fait que** ladite protéine de soie d'arachnides répond à la séquence polypeptidique SEQ ID N°3.

8. Composition cosmétique ou dermatologique selon la revendication 1, **caractérisée par le fait que** ledit analogue répond à l'une des séquences polypeptidiques SEQ ID N° 4, 5, 6, 7, 8, 9, 10 ou 11.

9. Composition cosmétique ou dermatologique selon l'une quelconque des revendications précédentes, **caractérisée par le fait que** ladite protéine de soies d'arachnides est présente en une proportion d'environ 10⁻⁴ à 30 % en poids par rapport au poids total de la composition.

10. Composition selon l'une quelconque des revendications précédentes, **caractérisée par le fait que** ladite composition est une composition anhydre contenant une phase grasse en une proportion de 10 à 90 % en poids par rapport au poids total de la composition, ladite phase grasse contenant au moins un corps gras liquide, solide ou semi-solide.

11. Composition selon la revendication 10, **caractérisée par le fait que** ledit corps gras est choisi dans le groupe constitué par l'isododécane, le polyisobutène hydrogéné, le squalane, l'isononanoate d'isononyle, les cyclotétra- et penta-diméthicones, la phényltriméthicone, les homopolymères d'éthylène, les copolymères d'éthylène et d'au moins un monomère répondant à la formule suivante :
CH₂=CH-R₃
dans laquelle :
R₃ représente un radical alkyle ayant de 1 à 30 atomes de carbone, un radical aryle ou aralkyle,
les cires microcristallines, l'ozokérite, la cire d'abeilles, la cire de candelilla et le propionate d'arachidyle.

12. Composition selon l'une quelconque des revendications précédentes, **caractérisée par le fait qu'**elle se présente sous la forme d'un gel huileux, d'une poudre compactée, d'une poudre coulée ou d'un stick.

13. Composition selon la revendication 12, **caractérisée par le fait que** le stick est un rouge à lèvres contenant au moins un colorant et/ou pigment en une proportion de 0,1 à 15 % en poids par rapport au poids total de la composition.

14. Composition selon l'une quelconque des revendications 1 à 9, **caractérisée par le fait qu'**elle se présente sous forme d'une dispersion stable du type eau-dans-l'huile ou huile-dans-l'eau, comprenant (i) une phase grasse en une proportion de 0,1 à 50 % en poids par rapport au poids total de la composition, ladite phase grasse contenant une protéine de soie d'arachnides naturelle, recombinante ou un analogue, en une proportion de 10⁻⁴ à 30 % en poids par rapport au poids total de la composition, (ii) une phase aqueuse en une proportion de 50 à 98,9 % en poids par rapport au poids total de la composition, et (iii) au moins un agent émulsionnant en une proportion de 1 à 10 % en poids par rapport au poids total de la composition.

15. Composition selon la revendication 14, **caractérisée par le fait que** ladite phase grasse contient au moins un corps gras tel que défini selon la revendication 11.

16. Composition selon l'une quelconque des revendications 1 à 9, **caractérisée par le fait qu'**elle se présente sous forme d'un vernis à ongles, incolore ou coloré, contenant :
(i) une protéine de soie d'arachnides en une proportion de 10⁻⁴ à 30 % en poids par rapport au poids total du vernis,
(ii) un mélange solvant pour vernis, et
(iii) une substance filmogène.

17. Composition selon la revendication 16, **caractérisée par le fait que** le mélange solvant est présent en une proportion de 55 à 90 % en poids et la substance filmogène en une proportion de 5 à 35 % en poids par rapport au poids total de la composition.

18. Composition selon l'une quelconque des revendications 1 à 9, **caractérisée par le fait que** ladite composition est une composition capillaire comprenant, dans un véhicule cosmétique choisi parmi les solutions aqueuses alcooliques et hydroalcooliques, une protéine de soie d'arachnides naturelle ou recombinante ou un analogue en une proportion de 10⁻⁴ à 30 % en poids par rapport au poids total de la composition.

19. Utilisation dans une composition cosmétique ou pour la préparation d'une composition dermatologique d'une quantité efficace d'au moins une protéine de soie d'arachnides naturelle ou recombinante ou un analogue d'une telle protéine, en tant qu'agent hydratant ou agent plastifiant et/ou agent filmogène rémanent à l'eau.

## Claims

1. Cosmetic or dermatological composition, **characterized in that** it contains an effective amount of at least one natural or recombinant spider silk protein or an analogue of such a protein and at least one conventional cosmetic adjuvant chosen from the group consisting of fillers, UVA and/or UVB sunscreehs, thixotropic agents, antioxidants, preserving agents, dyes, pigments, fragrances, thickeners, vitamins, hormones, moisturizers, wetting agents, anionic, nonionic or amphoteric polymers and dermatological active substances.

2. Cosmetic or dermatological composition according to Claim 1, **characterized in that** the said spider silk protein comprises an amino acid repeating unit of sequence (I) below:
[(XGG)_{w}(XGA)(GXG)ₓ(AGA)_{y}(G)_{z}AG]ₚ (I)
in which:
X corresponds to tyrosine or to glutamine,
G corresponds to glycine,
A corresponds to alanine,
w is an integer equal to 2 or 3,
x is an integer from 1 to 3,
y is an integer from 5 to 7,
z is an integer equal to 1 or 2, and
p is an integer such that the molecular weight of the protein is between 10 and 400 kDa.

3. Cosmetic or dermatological composition according to Claim 2, **characterized in that** the said spider silk protein corresponds to the peptide sequence SEQ ID No. 1.

4. Cosmetic or dermatological composition according to Claim 1, **characterized in that** the said spider silk protein comprises an amino acid repeating unit of sequence (II) below:
[(GPG₂YGPGQ₂)ₐ(X')₂S(A)_{b}]ₚ (II)
in which:
X' corresponds to the amino acid sequence GPS or GPG,
G corresponds to glycine,
A corresponds to alanine,
P corresponds to proline,
Y corresponds to tyrosine,
Q corresponds to glutamine,
S corresponds to serine,
a is equal to 2 or 3,
b is an integer from 7 to 10, and
p is an integer such that the molecular weight of the protein is between 10 and 400 kDa.

5. Cosmetic or dermatological composition according to Claim 4, **characterized in that** the said spider silk protein corresponds to the polypeptide sequence SEQ ID No. 2.

6. Cosmetic or dermatological composition according to Claim 1, **characterized in that** the said spider silk protein comprises an amino acid repeating unit of sequences (III) and/or (IV) below:
[(GR)(GA)ₗ(A)ₘ(GGX")ₙ(GA)ₗ(A)ₘ]ₚ (III)
and/or
[(GGX")ₙ(GA)ₘ(A)₁]ₚ (IV)
in which:
X" corresponds to tyrosine, glutamine or alanine,
G corresponds to glycine,
A corresponds to alanine,
R corresponds to arginine,
l is an integer from 1 to 6,
m is an integer from 0 to 4,
n is an integer ranging from 1 to 4, and
p is an integer such that the molecular weight of the protein is between 10 and 400 kDa.

7. Cosmetic or dermatological composition according to Claim 6, **characterized in that** the said spider silk protein corresponds to the polypeptide sequence SEQ ID No. 3.

8. Cosmetic or dermatological composition according to Claim 1, **characterized in that** the said analogue corresponds to one of the polypeptide sequences SEQ ID No. 4, 5, 6, 7, 8, 9, 10 or 11.

9. Cosmetic or dermatological composition according to any one of the preceding claims, **characterized in that** the said spider silk protein is present in a proportion of from about 10⁻⁴ to about 30% by weight relative to the total weight of the composition.

10. Composition according to any one of the preceding claims, **characterized in that** the said composition is an anhydrous composition containing a fatty phase in a proportion of from 10 to 90% by weight relative to the total weight of the composition, the said fatty phase containing at least one liquid, solid or semi-solid fatty substance.

11. Composition according to Claim 10, **characterized in that** the said fatty substance is chosen from the group consisting of isododecane, hydrogenated polyisobutene, squalane, isononyl isononanoate, cyclotetra- and -pentadimethicones, phenyltrimethicone, ethylene homopolymers, copolymers of ethylene and of at least one monomer corresponding to the following formula:
CH₂=CH-R₃
in which:
R₃ represents an alkyl radical containing from 1 to 30 carbon atoms or an aryl or aralkyl radical,
microcrystalline waxes, ozocerite, beeswax, candelilla wax and arachidyl propionate.

12. Composition according to any one of the preceding claims, **characterized in that** it is in the form of an oily gel, a compacted powder, a. cast powder or a stick.

13. Composition according to Claim 12, **characterized in that** the stick is a lipstick containing at least one dye and/or pigment in a proportion of from 0.1 to 15% by weight relative to the total weight of the composition.

14. Composition according to any one of Claims 1 to 9, **characterized in that** it is in the form of a stable dispersion of water-in-oil or oil-in-water type, comprising (i) a fatty phase in a proportion of from 0.1 to 50% by weight relative to the total weight of the composition, the said fatty phase containing a natural or recombinant spider silk protein, or an analogue, in a proportion of from 10⁻⁴ to 30% by weight relative to the total weight of the composition, (ii) an aqueous phase in a proportion of from 50 to 98.9% by weight relative to the total weight of the composition, and (iii) at least one emulsifier in a proportion of from 1 to 10% by weight relative to the total weight of the composition.

15. Composition according to Claim 14, **characterized in that** the said fatty phase contains at least one fatty substance as defined according to Claim 11.

16. Composition according to any one of Claims 1 to 9, **characterized in that** it is in the form of a colourless or coloured nail varnish containing:
(i) a spider silk protein in a proportion of from 10⁻⁴ to 30% by weight relative to the total weight of the varnish,
(ii) a solvent mixture for varnishes, and
(iii) a film-forming substance.

17. Composition according to Claim 16, **characterized in that** the solvent mixture is present in a proportion of from 55 to 90% by weight and the film-forming substance in a proportion of from 5 to 35% by weight relative to the total weight of the composition.

18. Composition according to any one of Claims 1 to 9, **characterized in that** the said composition is a hair composition comprising, in a cosmetic vehicle chosen from aqueous, alcoholic and aqueous-alcoholic solutions, a natural or recombinant spider silk protein or an analogue in a proportion of from 10⁻⁴ to 30% by weight relative to the total weight of the composition.

19. Use, in a cosmetic composition or for the preparation of a dermatological composition, of an effective amount of at least one natural or recombinant spider silk protein or an analogue of such a protein, as a moisturizer, plasticizer and/or water-resistant film-forming agent.

## Patentansprüche

1. Kosmetische oder dermatologische Zusammensetzung, **dadurch gekennzeichnet, dass** sie mindestens ein natürliches oder rekombinantes Seidenprotein von Arachniden oder ein Analogon eines solchen Proteins und mindestens einen herkömmlichen kosmetischen Zusatzstoff enthält, der unter den Füllstoffen, UV-A- und/oder UV-B-Lichtschutzfiltern, Thixotropiermitteln, Antioxidantien, Konservierungsmitteln, Farbstoffen, Pigmenten, Parfums, Verdickungsmitteln, Vitaminen, Hormonen, Hydratisierungsmitteln, Feuchthaltemitteln, anionischen Polymeren, nichtionischen Polymeren oder amphoteren Polymeren und dermatologischen Wirkstoffen ausgewählt ist.

2. Kosmetische oder dermatologische Zusammensetzung nach Anspruch 1, **dadurch gekennzeichnet, dass** das Seidenprotein von Arachniden eine repetitive Aminosäureeinheit der folgenden Sequenz (I) enthält:
[(XGG)_{w}(XGA)(GXG)ₓ(AGA)_{y}(G)_{z}AG]ₚ (I),
worin:
X Tyrosin oder Glutamin entspricht,
G Glycin entspricht,
A Alanin entspricht,
w eine ganze Zahl gleich 2 oder 3 ist,
x eine ganze Zahl im Bereich von 1 bis 3 ist,
y eine ganze Zahl von 5 bis 7 ist,
z eine ganze Zahl gleich 1 oder 2, und
p eine solche ganze Zahl ist, dass die Molekularmasse des Proteins im Bereich von 10 bis 400 kDa liegt.

3. Kosmetische oder dermatologische Zusammensetzung nach Anspruch 2, **dadurch gekennzeichnet, dass** das Seidenprotein von Arachniden der Peptidsequenz SEQ ID Nr. 1 entspricht.

4. Kosmetische oder dermatologische Zusammensetzung nach Anspruch 1, **dadurch gekennzeichnet, dass** das Seidenprotein von Arachniden eine repetitive Aminosäuresequenz der folgenden Sequenz (II) enthält:
[(GPG₂YGPGQ₂)ₐ(X')₂S(A)_{b}]ₚ (II),
worin:
X' der Aminosäuresequenz GPS oder GPG entspricht,
G Glycin entspricht,
A Alanin entspricht,
P Prolin entspricht,
Y Tyrosin entspricht,
Q Glutamin entspricht,
S Serin entspricht,
a 2 oder 3 bedeutet,
b eine ganze Zahl von 7 bis 10 ist, und
p eine solche ganze Zahl bedeutet, dass die Molekularmasse des Proteins im Bereich von 10 bis 400 kDa liegt.

5. Kosmetische oder dermatologische Zusammensetzung nach Anspruch 4, **dadurch gekennzeichnet, dass** das Seidenprotein von Arachniden der Polypeptidsequenz SEQ ID Nr. 2 entspricht.

6. Kosmetische oder dermatologische Zusammensetzung nach Anspruch 1, **dadurch gekennzeichnet, dass** das Seidenprotein von Arachniden eine repetitive Aminosäuresequenz der folgenden Sequenzen (III) und/oder (IV) enthält:
[(GR)(GA)ₗ(A)ₘ(GGX")ₙ(GA)ₗ(A)ₘ]ₚ (III),
und/oder
[(GGX")ₙ(GA)ₘ(A)ₗ]ₚ (IV),
wobei:
X" Tyrosin, Glutamin oder Alanin entspricht,
G Glycin entspricht,
A Alanin entspricht,
R Arginin entspricht,
l eine ganze Zahl von 1 bis 6 ist,
m 0 oder eine ganze Zahl von 1 bis 4 bedeutet,
n eine ganze Zahl von 1 bis 4 ist, und
p eine solche ganze Zahl bedeutet, dass die Molekularmasse des Proteins im Bereichs von 10 bis 400 kDa liegt.

7. Kosmetische oder dermatologische Zusammensetzung nach Anspruch 6, **dadurch gekennzeichnet, dass** das Seidenprotein von Arachniden der Polypeptidsequenz SEQ ID Nr. 3 entspricht.

8. Kosmetische oder dermatologische Zusammensetzung nach Anspruch 1, **dadurch gekennzeichnet, dass** die analoge Verbindung einer der Polypeptidsequenzen SEQ ID Nr. 4, 5, 6, 7, 8, 9, 10 oder 11 entspricht.

9. Kosmetische oder dermatologische Zusammensetzung nach einem der vorhergehenden Ansprüche, **dadurch gekennzeichnet, dass** das Seidenprotein von Arachniden in einem Mengenanteil von etwa 10⁻⁴ bis 30 Gew.-%, bezogen auf das Gesamtgewicht der Zusammensetzung, vorliegt.

10. Zusammensetzung nach einem der vorhergehenden Ansprüche, **dadurch gekennzeichnet, dass** die Zusammensetzung eine wasserfreie Zusammensetzung ist, die eine Fettphase in einer Menge von 10 bis 90 Gew.-%, bezogen auf das Gesamtgewicht der Zusammensetzung, enthält, wobei die Fettphase mindestens eine flüssige, feste oder halbfeste Fettsubstanz enthält.

11. Zusammensetzung nach Anspruch 10, **dadurch gekennzeichnet, dass** die Fettsubstanz unter Isododecan, hydriertem Polyisobuten, Squalan, Isononylisononanoat, Cyclotetra- und Pentadimethiconen, Phenyltrimethicon, Ethylenhomopolymeren, Copolymeren von Ethylen und mindestens einem Monomer der folgenden Formel:
CH₂=CH-R₃,
worin R₃ eine Alkylgruppe mit 1 bis 30 Kohlenstoffatomen, Aryl oder Aralkyl bedeutet,
monokristallinen Wachsen, Ozokerit, Bienenwachs, Candelillawachs und Arachidylpropionat ausgewählt ist.

12. Zusammensetzung nach einem der vorhergehenden Ansprüche, **dadurch gekennzeichnet, dass** sie als öliges Gel, gepresstes Pulver, gegossenes Pulver oder Stift vorliegt.

13. Zusammensetzung nach Anspruch 12, **dadurch gekennzeichnet, dass** der Stift ein Lippenstift ist, der mindestens einen Farbstoff und/oder mindestens ein Pigment in einer Menge von 0,1 bis 15 Gew.-%, bezogen auf das Gesamtgewicht der Zusammensetzung, enthält.

14. Zusammensetzung nach einem der Ansprüche 1 bis 9, **dadurch gekennzeichnet, dass** sie als stabile Dispersion vom Typ Wasser-in-Öl oder Öl-in-Wasser vorliegt, die (i) eine Fettphase in einer Menge von etwa 0,1 bis 50 Gew.-%, bezogen auf das Gesamtgewicht der Zusammensetzung, wobei die Fettphase ein natürliches oder rekombinantes Seidenprotein oder ein analoges Protein von Arachniden in einer Menge von etwa 10⁻⁴ bis 30 Gew.-%, bezogen auf das Gesamtgewicht der Zusammensetzung, enthält, (ii) eine wässrige Phase in einer Menge von etwa 50 bis 98,9 Gew.-%, bezogen auf das Gesamtgewicht der Zusammensetzung, und (iii) mindestens einen Emulgator in einer Menge von 1 bis 10 Gew.-%, bezogen auf das Gesamtgewicht der Zusammensetzung, enthält.

15. Zusammensetzung nach Anspruch 14, **dadurch gekennzeichnet, dass** die Fettphase mindestens eine Fettsubstanz nach Anspruch 11 enthält.

16. Zusammensetzung nach einem der Ansprüche 1 bis 9, **dadurch gekennzeichnet, dass** sie als farbloser oder farbiger Nagellack vorliegt, der enthält:
(i) ein Seidenprotein von Arachniden in einer Menge von 10⁻⁴ bis 30 Gew.-%, bezogen auf das Gesamtgewicht der Zusammensetzung,
(ii) ein Lösungsmittelgemisch für den Lack und
(iii) eine filmbildende Substanz.

17. Zusammensetzung nach Anspruch 16, **dadurch gekennzeichnet, dass** das Lösungsmittelgemisch in einer Menge von 55 bis 90 Gew.-% und die filmbildende Substanz in einer Menge von 5 bis 35 Gew.-%, bezogen auf das Gesamtgewicht der Zusammensetzung, enthalten ist.

18. Zusammensetzung nach einem der Ansprüche 1 bis 9, **dadurch gekennzeichnet, dass** die Zusammensetzung eine Zusammensetzung für die Haarbehandlung ist, die in einem kosmetischen Träger, der unten den wässrigen, alkoholischen und wässrigalkoholischen Lösungen ausgewählt ist, ein natürliches oder rekombinantes Seidenprotein von Arachniden oder an Analogon davon in einer Menge von 10⁻⁴ bis 30 Gew.-%, bezogen auf das Gesamtgewicht der Zusammensetzung, enthält.

19. Verwendung mindestens eines natürlichen oder rekombinanten Seidenproteins von Arachniden oder eines Analogons eines solchen Proteins in einer wirksamen Menge in einer kosmetischen Zusammensetzung oder zur Herstellung einer dermatologischen Zusammensetzung als Hydratisierungsmittel oder Weichmacher und/oder wasserbeständiger Filmbildner.


### LISTE DE SEQUENCES

(1) INFORMATIONS GENERALES:
   (i) DEPOSANT:
      (A) NOM: L'OREAL
      (B) RUE: 14, rue Royale
      (C) VILLE: PARIS
      (E) PAYS: FRANCE
      (F) CODE POSTAL: 75008
   (ii) TITRE DE L' INVENTION: Composition cosmetique ou dermatologique contenant au moins une proteine de soie d'arachnides naturelle recombinante ou analogue
   (iii) NOMBRE DE SEQUENCES: 11
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 651 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE :
      (A) ORGANISME: Nephila clavipes
   (xi) DESCRIPTION DE LA SEQUENCE: SEQ ID NO: 1:
   Gln Gly Ala Gly Ala Ala Ala Ala Ala Ala Gly Gly Ala Gly Gln Gly
   1               5                   10                  15
   Gly Tyr Gly Gly Leu Gly Gly Gln Gly Ala Gly Gln Gly Gly Tyr Gly
   20 25 30
   Gly Leu Gly Gly Gln Gly Ala Gly Gln Gly Ala Gly Ala Ala Ala Ala
   35 40 45
   Ala Ala Ala Gly Gly Ala Gly Gln Gly Gly Tyr Gly Gly Leu Gly Ser
   50 55 60
   Gln Gly Ala Gly Arg Gly Gly Gln Gly Ala Gly Ala Ala Ala Ala Ala
   65 70 75 80
   Ala Gly Gly Ala Gly Gln Gly Gly Tyr Gly Gly Leu Gly Ser Gln Gly
   85 90 95
   Ala Gly Arg Gly Gly Leu Gly Gly Gln Gly Ala Gly Ala Ala Ala Ala
   100 105 110
   Ala Ala Ala Gly Gly Ala Gly Gln Gly Gly Tyr Gly Gly Leu Gly Asn
   115 120 125
   Gln Gly Ala Gly Arg Gly Gly Gln Gly Ala Ala Ala Ala Ala Ala Gly
   130 135 140
   Gly Ala Gly Gln Gly Gly Tyr Gly Gly Leu Gly Ser Gln Gly Ala Gly
   145 150 155 160
   Arg Gly Gly Leu Gly Gly Gln Gly Ala Gly Ala Ala Ala Ala Ala Ala
   165 170 175
   Gly Gly Ala Gly Gln Gly Gly Tyr Gly Gly Leu Gly Gly Gln Gly Ala
   180 185 190
   Gly Gln Gly Gly Tyr Gly Gly Leu Gly Ser Gln Gly Ala Gly Arg Gly
   195 200 205
   Gly Leu Gly Gly Gln Gly Ala Gly Ala Ala Ala Ala Ala Ala Ala Gly
   210 215 220
   Gly Ala Gly Gln Gly Gly Leu Gly Gly Gln Gly Ala Gly Gln Gly Ala
   225 230 235 240
   Gly Ala Ser Ala Ala Ala Ala Gly Gly Ala Gly Gln Gly Gly Tyr Gly
   245 250 255
   Gly Leu Gly Ser Gln Gly Ala Gly Arg Gly Gly Glu Gly Ala Gly Ala
   260 265 270
   Ala Ala Ala Ala Ala Gly Gly Ala Gly Gln Gly Gly Tyr Gly Gly Leu
   275 280 285
   Gly Gly Gln Gly Ala Gly Gln Gly Gly Tyr Gly Gly Leu Gly Ser Gln
   290 295 300
   Gly Ala Gly Arg Gly Gly Leu Gly Gly Gln Gly Ala Gly Ala Ala Ala
   305 310 315 320
   Ala Gly Gly Ala Gly Gln Gly Gly Leu Gly Gly Gln Gly Ala Gly Gln
   325 330 335
   Gly Ala Gly Ala Ala Ala Ala Ala Ala Gly Gly Ala Gly Gln Gly Gly
   340 345 350
   Tyr Gly Gly Leu Gly Ser Gln Gly Ala Gly Arg Gly Gly Leu Gly Gly
   355 360 365
   Gln Gly Ala Gly Ala Val Ala Ala Ala Ala Ala Gly Gly Ala Gly Gln
   370 375 380
   Gly Gly Tyr Gly Gly Leu Gly Ser Gln Gly Ala Gly Arg Gly Gly Gln
   385 390 395 400
   Gly Ala Gly Ala Ala Ala Ala Ala Ala Gly Gly Ala Gly Gln Arg Gly
   405 410 415
   Tyr Gly Gly Leu Gly Asn Gln Gly Ala Gly Arg Gly Gly Leu Gly Gly
   420 425 430
   Gln Gly Ala Gly Ala Ala Ala Ala Ala Ala Ala Gly Gly Ala Gly Gln
   435 440 445
   Gly Gly Tyr Gly Gly Leu Gly Asn Gln Gly Ala Gly Arg Gly Gly Gln
   450 455 460
   Gly Ala Ala Ala Ala Ala Gly Gly Ala Gly Gln Gly Gly Tyr Gly Gly
   465 470 475 480
   Leu Gly Ser Gln Gly Ala Gly Arg Gly Gly Gln Gly Ala Gly Ala Ala
   485 490 495
   Ala Ala Ala Ala Val Gly Ala Gly Gln Glu Gly Ile Arg Gly Gln Gly
   500 505 510
   Ala Gly Gln Gly Gly Tyr Gly Gly Leu Gly Ser Gln Gly Ser Gly Arg
   515 520 525
   Gly Gly Leu Gly Gly Gln Gly Ala Gly Ala Ala Ala Ala Ala Ala Gly
   530 535 540
   Gly Ala Gly Gln Gly Gly Leu Gly Gly Gln Gly Ala Gly Gln Gly Ala
   545 550 555 560
   Gly Ala Ala Ala Ala Ala Ala Gly Gly Val Arg Gln Gly Gly Tyr Gly
                   565                 570                 575
   Gly Leu Gly Ser Gln Gly Ala Gly Arg Gly Gly Gln Gly Ala Gly Ala
               580                 585                 590
   Ala Ala Ala Ala Ala Gly Gly Ala Gly Gln Gly Gly Tyr Gly Gly Leu
           595                 600                 605
   Gly Gly Gln Gly Val Gly Arg Gly Gly Leu Gly Gly Gln Gly Ala Gly
       610                 615                 620
   Ala Ala Ala Ala Gly Gly Ala Gly Gln Gly Gly Tyr Gly Gly Val Gly
   625                 630                 635                 640
   Ser Gly Ala Ser Ala Ala Ser Ala Ala Ala Ala
                   645                 650
(2) INFORMATIONS POUR LA SEQ ID NO: 2:
   (i) CARACTERISTIQUES DE LA SEQUENCE:
      (A) LONGUEUR: 531 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 2:
   Pro Gly Gly Tyr Gly Pro Gly Gln Gln Gly Pro Gly Gly Tyr Gly Pro
   1               5                   10                  15
   Gly Gln Gln Gly Pro Ser Gly Pro Gly Ser Ala Ala Ala Ala Ala Ala
               20                  25                  30
   Ala Ala Ala Ala Gly Pro Gly Gly Tyr Gly Pro Gly Gln Gln Gly Pro
           35                  40                  45
   Gly Gly Tyr Gly Pro Gly Gln Gln Gly Pro Gly Arg Tyr Gly Pro Gly
   50 55 60
   Gln Gln Gly Pro Ser Gly Pro Gly Ser Ala Ala Ala Ala Ala Ala Gly
   65 70 75 80
   Ser Gly Gln Gln Gly Pro Gly Gly Tyr Gly Pro Arg Gln Gln Gly Pro
   85 90 95
   Gly Gly Tyr Gly Gln Gly Gln Gln Gly Pro Ser Gly Pro Gly Ser Ala
   100 105 110
   Ala Ala Ala Ser Ala Ala Ala Ser Ala Glu Ser Gly Gln Gln Gly Pro
   115 120 125
   Gly Gly Tyr Gly Pro Gly Gln Gln Gly Pro Gly Gly Tyr Gly Pro Gly
   130 135 140
   Gln Gln Gly Pro Gly Gly Tyr Gly Pro Gly Gln Gln Gly Pro Ser Gly
   145 150 155 160
   Pro Gly Ser Ala Ala Ala Ala Ala Ala Ala Ala Ser Gly Pro Gly Gln
   165 170 175
   Gln Gly Pro Gly Gly Tyr Gly Pro Gly Gln Gln Gly Pro Gly Gly Tyr
   180 185 190
   Gly Pro Gly Gln Gln Gly Pro Ser Gly Pro Gly Ser Ala Ala Ala Ala
   195 200 205
   Ala Ala Ala Ala Ser Gly Pro Gly Gln Gln Gly Pro Gly Gly Tyr Gly
   210 215 220
   Pro Gly Gln Gln Gly Pro Gly Gly Tyr Gly Pro Gly Gln Gln Gly Leu
   225 230 235 240
   Ser Gly Pro Gly Ser Ala Ala Ala Ala Ala Ala Ala Gly Pro Gly Gln
   245 250 255
   Gln Gly Pro Gly Gly Tyr Gly Pro Gly Gln Gln Gly Pro Ser Gly Pro
   260 265 270
   Gly Ser Ala Ala Ala Ala Ala Ala Ala Ala Ala Gly Pro Gly Gly Tyr
   275 280 285
   Gly Pro Gly Gln Gln Gly Pro Gly Gly Tyr Gly Pro Gly Gln Gln Gly
   290 295 300
   Pro Ser Gly Ala Gly Ser Ala Ala Ala Ala Ala Ala Ala Gly Pro Gly
   305 310 315 320
   Gln Gln Gly Leu Gly Gly Tyr Gly Pro Gly Gln Gln Gly Pro Gly Gly
                       325                     330                     335
   Tyr Gly Pro Gly Gln Gln Gly Pro Gly Gly Tyr Gly Pro Gly Ser Ala
                   340                     345                     350
   Ser Ala Ala Ala Ala Ala Ala Gly Pro Gly Gln Gln Gly Pro Gly Gly
               355                     360                     365
   Tyr Gly Pro Gly Gln Gln Gly Pro Ser Gly Pro Gly Ser Ala Ser Ala
           370                     375                     380
   Ala Ala Ala Ala Ala Ala Ala Gly Pro Gly Gly Tyr Gly Pro Gly Gln
   385                     390                     395                     400
   Gln Gly Pro Gly Gly Tyr Ala Pro Gly Gln Gln Gly Pro Ser Gly Pro
                       405                     410                     415
   Gly Ser Ala Ser Ala Ala Ala Ala Ala Ala Ala Ala Gly Pro Gly Gly
                   420                     425                     430
   Tyr Gly Pro Gly Gln Gln Gly Pro Gly Gly Tyr Ala Pro Gly Gln Gln
               435                     440                     445
   Gly Pro Ser Gly Pro Gly Ser Ala Ala Ala Ala Ala Ala Ala Ser Ala
       450                     455                     460
   Gly Pro Gly Gly Tyr Gly Pro Ala Gln Gln Gly Pro Ser Gly Pro Gly
   465                     470                     475                     480
   Ile Ala Ala Ser Ala Ala Ser Ala Gly Pro Gly Gly Tyr Gly Pro Ala
                       485                     490                     495
   Gln Gln Gly Pro Ala Gly Tyr Gly Pro Gly Ser Ala Val Ala Ala Ser
                   500                     505                     510
   Ala Gly Ala Gly Ser Ala Gly Tyr Gly Pro Gly Ser Gln Ala Ser Ala
               515                     520                     525
   Ala Ala Ser
           530
(2) INFORMATIONS POUR LA SEQ ID NO: 3:
   (i) CARACTERISTIQUES DE LA SEQUENCE:
      (A) LONGUEUR: 616 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 3:
   Gly Ala Ala Gly Ala Gly Gly Tyr Gly Arg Gly Ala Gly Gly Tyr Gly
   1 5 10 15
   Gly Gln Gly Gly Tyr Gly Ala Gly Ala Gly Ala Gly Ala Ala Ala Ala
   20 25 30
   Ala Gly Ala Gly Ala Gly Gly Ala Gly Gly Tyr Gly Arg Gly Ala Gly
   35 40 45
   Ala Gly Ala Gly Ala Ala Ala Gly Ala Gly Ala Gly Ala Gly Gly Ala
   50 55 60
   Gly Tyr Gly Gly Gln Gly Gly Tyr Gly Ala Gly Ala Gly Ala Gly Ala
   65 70 75 80
   Ala Ala Ala Ala Gly Ala Gly Ala Gly Gly Ala Gly Gly Tyr Gly Arg
   85 90 95
   Gly Ala Gly Ala Gly Ala Gly Ala Ala Ala Gly Ala Gly Ala Gly Gly
   100 105 110
   Tyr Gly Gly Gln Gly Gly Tyr Gly Ala Gly Ala Gly Ala Gly Ala Ala
   115 120 125
   Ala Ala Ala Ala Gly Ala Gly Ser Gly Gly Ala Gly Gly Tyr Gly Arg
   130 135 140
   Gly Ala Gly Ala Gly Ala Gly Ala Ala Ala Gly Ala Gly Ala Cys Ala
   145 150 155 160
   Gly Ser Tyr Gly Gly Gln Gly Gly Tyr Gly Ala Gly Ala Gly Ala Gly
   165 170 175
   Ala Ala Ala Ala Ala Gly Ala Gly Ala Gly Gly Ala Gly Gly Tyr Gly
   180 185 190
   Arg Gly Ala Gly Ala Gly Ala Gly Ala Gly Ala Gly Ala Ala Ala Arg
   195 200 205
   Ala Gly Ala Gly Ala Gly Gly Ala Ala Ala Ala Ala Gly Ala Gly Ala
   210 215 220
   Gly Gly Ala Gly Gly Tyr Gly Arg Gly Ala Gly Ala Gly Ala Gly Ala
   225 230 235 240
   Ala Ala Gly Ala Gly Ala Gly Ala Gly Gly Tyr Gly Gly Gln Ser Gly
   245 250 255
   Tyr Gly Ala Gly Ala Gly Ala Ala Ala Ala Ala Gly Ala Gly Ala Gly
   260 265 270
   Gly Ala Gly Gly Tyr Gly Arg Gly Ala Gly Ala Gly Ala Gly Ala Ala
   275 280 285
   Ala Gly Ala Gly Ala Gly Ala Ala Ala Gly Ala Gly Ala Gly Gly Tyr
   290 295 300
   Gly Gly Gln Gly Gly Tyr Gly Ala Gly Ala Gly Ala Gly Ala Ala Ala
   305 310 315 320
   Ala Ala Gly Ala Gly Ala Gly Gly Ala Gly Gly Tyr Gly Arg Gly Ala
   325 330 335
   Gly Ala Gly Ala Gly Ala Ala Ala Gly Ala Gly Ala Gly Gly Tyr Gly
   340 345 350
   Gly Gln Gly Gly Tyr Gly Ala Gly Ala Gly Ala Gly Ala Ala Ala Ala
   355 360 365
   Ala Thr Gly Ala Gly Gly Ala Gly Gly Tyr Gly Arg Gly Ala Gly Ala
   370 375 380
   Gly Ala Gly Ala Ala Ala Gly Ala Gly Ala Gly Thr Gly Gly Ala Gly
   385 390 395 400
   Tyr Gly Gly Gln Gly Gly Tyr Gly Ala Gly Ala Gly Ala Gly Ala Ala
   405 410 415
   Ala Ala Ala Gly Ala Gly Ala Gly Gly Ala Gly Tyr Gly Arg Gly Ala
   420 425 430
   Gly Ala Gly Ala Gly Ala Ala Ala Gly Ala Gly Ala Gly Ala Ala Ala
   435 440 445
   Gly Ala Gly Ala Gly Ala Gly Gly Tyr Gly Gly Gln Gly Gly Tyr Gly
   450 455 460
   Ala Gly Ala Arg Ala Gly Ala Ala Ala Ala Ala Gly Ala Gly Ala Gly
   465 470 475 480
   Gly Ala Ala Gly Tyr Ser Arg Gly Gly Arg Ala Gly Ala Ala Gly Ala
                   485                 490                 495
   Gly Ala Gly Ala Ala Ala Gly Ala Gly Ala Gly Ala Gly Gly Tyr Gly
               500                 505                 510
   Gly Gln Gly Gly Tyr Gly Ala Gly Ala Gly Ala Gly Ala Ala Ala Ala
           515                 520                 525
   Ala Gly Ala Gly Ser Gly Gly Ala Gly Gly Tyr Gly Arg Gly Ala Gly
       530                 535                 540
   Ala Gly Ala Ala Ala Gly Ala Gly Ala Ala Ala Gly Ala Gly Ala Gly
   545                 550                 555                 560
   Ala Gly Gly Tyr Gly Gly Gln Gly Gly Tyr Gly Ala Gly Ala Gly Ala
                   565                 570                 575
   Ala Ala Ala Ala Gly Ala Gly Ala Gly Arg Gly Gly Tyr Gly Arg Gly
               580                 585                 590
   Ala Gly Ala Gly Gly Tyr Gly Gly Gln Gly Gly Tyr Gly Ala Gly Ala
           595                 600                 605
   Gly Ala Gly Ala Ala Ala Ala Ala
       610                 615
(2) INFORMATIONS POUR LA SEQ ID NO: 4:
   (i) CARACTERISTIQUES DE LA SEQUENCE:
      (A) LONGUEUR: 101 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 4:
   Gly Ala Gly Arg Gly Gly Gln Gly Ala Gly Ala Ala Ala Ala Ala Ala
   1               5                   10                  15
   Gly Gly Ala Gly Gln Gly Gly Tyr Gly Gly Leu Gly Ser Gln Gly Ala
               20                  25                  30
   Gly Arg Gly Gly Leu Gly Gly Gln Gly Ala Gly Ala Ala Ala Ala Ala
           35                  40                  45
   Ala Ala Gly Gly Ala Gly Gln Gly Gly Leu Gly Ser Gln Gly Ala Gly
       50                  55                  60
   Gln Gly Ala Gly Ala Ala Ala Ala Ala Ala Gly Gly Ala Gly Gln Gly
   65                  70                  75                  80
   Gly Tyr Gly Gly Leu Gly Ser Gln Gly Ala Gly Gln Gly Gly Tyr Gly
                   85                  90                  95
   Gly Leu Gly Ser Gln
               100
(2) INFORMATIONS POUR LA SEQ ID NO: 5:
   (i) CARACTERISTIQUES DE LA SEQUENCE:
      (A) LONGUEUR: 606 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 5:
   Gly Ala Gly Arg Gly Gly Gln Gly Ala Gly Ala Ala Ala Ala Ala Ala
   1               5                   10                  15
   Gly Gly Ala Gly Gln Gly Gly Tyr Gly Gly Leu Gly Ser Gln Gly Ala
               20                  25                  30
   Gly Arg Gly Gly Leu Gly Gly Gln Gly Ala Gly Ala Ala Ala Ala Ala
           35                  40                  45
   Ala Ala Gly Gly Ala Gly Gln Gly Gly Leu Gly Ser Gln Gly Ala Gly
       50                  55                  60
   Gln Gly Ala Gly Ala Ala Ala Ala Ala Ala Gly Gly Ala Gly Gln Gly
   65                  70                  75                  80
   Gly Tyr Gly Gly Leu Gly Ser Gln Gly Ala Gly Gln Gly Gly Tyr Gly
                   85                  90                  95
   Gly Leu Gly Ser Gln Gly Ala Gly Arg Gly Gly Gln Gly Ala Gly Ala
               100                 105                 110
   Ala Ala Ala Ala Ala Gly Gly Ala Gly Gln Gly Gly Tyr Gly Gly Leu
           115                 120                 125
   Gly Ser Gln Gly Ala Gly Arg Gly Gly Leu Gly Gly Gln Gly Ala Gly
       130                 135                 140
   Ala Ala Ala Ala Ala Ala Ala Gly Gly Ala Gly Gln Gly Gly Leu Gly
   145                 150                 155                 160
   Ser Gln Gly Ala Gly Gln Gly Ala Gly Ala Ala Ala Ala Ala Ala Gly
                   165                 170                 175
   Gly Ala Gly Gln Gly Gly Tyr Gly Gly Leu Gly Ser Gln Gly Ala Gly
               180                 185                 190
   Gln Gly Gly Tyr Gly Gly Leu Gly Ser Gln Gly Ala Gly Arg Gly Gly
           195                 200                 205
   Gln Gly Ala Gly Ala Ala Ala Ala Ala Ala Gly Gly Ala Gly Gln Gly
       210                 215                 220
   Gly Tyr Gly Gly Leu Gly Ser Gln Gly Ala Gly Arg Gly Gly Leu Gly
   225                 230                 235                 240
   Gly Gln Gly Ala Gly Ala Ala Ala Ala Ala Ala Ala Gly Gly Ala Gly
                   245                 250                 255
   Gln Gly Gly Leu Gly Ser Gln Gly Ala Gly Gln Gly Ala Gly Ala Ala
               260                 265                 270
   Ala Ala Ala Ala Gly Gly Ala Gly Gln Gly Gly Tyr Gly Gly Leu Gly
           275                 280                 285
   Ser Gln Gly Ala Gly Gln Gly Gly Tyr Gly Gly Leu Gly Ser Gln Gly
       290                 295                 300
   Ala Gly Arg Gly Gly Gln Gly Ala Gly Ala Ala Ala Ala Ala Ala Gly
   305                 310                 315                 320
   Gly Ala Gly Gln Gly Gly Tyr Gly Gly Leu Gly Ser Gln Gly Ala Gly
                   325                 330                 335
   Arg Gly Gly Leu Gly Gly Gln Gly Ala Gly Ala Ala Ala Ala Ala Ala
               340                 345                 350
   Ala Gly Gly Ala Gly Gln Gly Gly Leu Gly Ser Gln Gly Ala Gly Gln
           355                 360                 365
   Gly Ala Gly Ala Ala Ala Ala Ala Ala Gly Gly Ala Gly Gln Gly Gly
       370                 375                 380
   Tyr Gly Gly Leu Gly Ser Gln Gly Ala Gly Gln Gly Gly Tyr Gly Gly
   385                 390                 395                 400
   Leu Gly Ser Gln Gly Ala Gly Arg Gly Gly Gln Gly Ala Gly Ala Ala
                   405                 410                 415
   Ala Ala Ala Ala Gly Gly Ala Gly Gln Gly Gly Tyr Gly Gly Leu Gly
               420                 425                 430
   Ser Gln Gly Ala Gly Arg Gly Gly Leu Gly Gly Gln Gly Ala Gly Ala
           435                 440                 445
   Ala Ala Ala Ala Ala Ala Gly Gly Ala Gly Gln Gly Gly Leu Gly Ser
       450                 455                 460
   Gln Gly Ala Gly Gln Gly Ala Gly Ala Ala Ala Ala Ala Ala Gly Gly
   465                 470                 475                 480
   Ala Gly Gln Gly Gly Tyr Gly Gly Leu Gly Ser Gln Gly Ala Gly Gln
                   485                 490                 495
   Gly Gly Tyr Gly Gly Leu Gly Ser Gln Gly Ala Gly Arg Gly Gly Gln
               500                 505                 510
   Gly Ala Gly Ala Ala Ala Ala Ala Ala Gly Gly Ala Gly Gln Gly Gly
           515                 520                 525
   Tyr Gly Gly Leu Gly Ser Gln Gly Ala Gly Arg Gly Gly Leu Gly Gly
       530                 535                 540
   Gln Gly Ala Gly Ala Ala Ala Ala Ala Ala Ala Gly Gly Ala Gly Gln
   545                 550                 555                 560
   Gly Gly Leu Gly Ser Gln Gly Ala Gly Gln Gly Ala Gly Ala Ala Ala
                   565                 570                 575
   Ala Ala Ala Gly Gly Ala Gly Gln Gly Gly Tyr Gly Gly Leu Gly Ser
                       580                 585                 590
   Gln Gly Ala Gly Gln Gly Gly Tyr Gly Gly Leu Gly Ser Gln
           595                 600                 605
(2) INFORMATIONS POUR LA SEQ ID NO: 6:
   (i) CARACTERISTIQUES DE LA SEQUENCE:
      (A) LONGUEUR: 101 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON )
   (iv) ANTI-SENS : NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 6:
   Ser Gln Gly Ala Gly Gln Gly Gly Tyr Gly Gly Leu Gly Ser Gln Gly
   1               5                   10                  15
   Ala Gly Arg Gly Gly Leu Gly Gly Gln Gly Ala Gly Ala Ala Ala Ala
               20                  25                  30
   Ala Ala Ala Gly Gly Ala Gly Gln Gly Gly Leu Gly Ser Gln Gly Ala
           35                  40                  45
   Gly Gln Gly Ala Gly Ala Ala Ala Ala Ala Ala Gly Gly Ala Gly Gln
       50                  55                  60
   Gly Gly Tyr Gly Gly Leu Gly Ser Gln Gly Ala Gly Arg Gly Gly Gln
   65                  70                  75                  80
   Gly Ala Gly Ala Ala Ala Ala Ala Ala Gly Gly Ala Gly Gln Gly Gly
                   85                  90                  95
   Tyr Gly Gly Leu Gly
               100
(2) INFORMATIONS POUR LA SEQ ID NO: 7:
   (i) CARACTERISTIQUES DE LA SEQUENCE:
      (A) LONGUEUR: 606 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 7:
   Ser Gln Gly Ala Gly Gln Gly Gly Tyr Gly Gly Leu Gly Ser Gln Gly
   1 5 10 15
   Ala Gly Arg Gly Gly Leu Gly Gly Gln Gly Ala Gly Ala Ala Ala Ala
   20 25 30
   Ala Ala Ala Gly Gly Ala Gly Gln Gly Gly Leu Gly Ser Gln Gly Ala
   35 40 45
   Gly Gln Gly Ala Gly Ala Ala Ala Ala Ala Ala Gly Gly Ala Gly Gln
   50 55 60
   Gly Gly Tyr Gly Gly Leu Gly Ser Gln Gly Ala Gly Arg Gly Gly Gln
   65 70 75 80
   Gly Ala Gly Ala Ala Ala Ala Ala Ala Gly Gly Ala Gly Gln Gly Gly
   85 90 95
   Tyr Gly Gly Leu Gly Ser Gln Gly Ala Gly Gln Gly Gly Tyr Gly Gly
   100 105 110
   Leu Gly Ser Gln Gly Ala Gly Arg Gly Gly Leu Gly Gly Gln Gly Ala
   115 120 125
   Gly Ala Ala Ala Ala Ala Ala Ala Gly Gly Ala Gly Gln Gly Gly Leu
   130 135 140
   Gly Ser Gln Gly Ala Gly Gln Gly Ala Gly Ala Ala Ala Ala Ala Ala
   145 150 155 160
   Gly Gly Ala Gly Gln Gly Gly Tyr Gly Gly Leu Gly Ser Gln Gly Ala
   165 170 175
   Gly Arg Gly Gly Gln Gly Ala Gly Ala Ala Ala Ala Ala Ala Gly Gly
   180 185 190
   Ala Gly Gln Gly Gly Tyr Gly Gly Leu Gly Ser Gln Gly Ala Gly Gln
   195 200 205
   Gly Gly Tyr Gly Gly Leu Gly Ser Gln Gly Ala Gly Arg Gly Gly Leu
   210 215 220
   Gly Gly Gln Gly Ala Gly Ala Ala Ala Ala Ala Ala Ala Gly Gly Ala
   225 230 235 240
   Gly Gln Gly Gly Leu Gly Ser Gln Gly Ala Gly Gln Gly Ala Gly Ala
   245 250 255
   Ala Ala Ala Ala Ala Gly Gly Ala Gly Gln Gly Gly Tyr Gly Gly Leu
   260 265 270
   Gly Ser Gln Gly Ala Gly Arg Gly Gly Gln Gly Ala Gly Ala Ala Ala
   275 280 285
   Ala Ala Ala Gly Gly Ala Gly Gln Gly Gly Tyr Gly Gly Leu Gly Ser
   290 295 300
   Gln Gly Ala Gly Gln Gly Gly Tyr Gly Gly Leu Gly Ser Gln Gly Ala
   305 310 315 320
   Gly Arg Gly Gly Leu Gly Gly Gln Gly Ala Gly Ala Ala Ala Ala Ala
   325 330 335
   Ala Ala Gly Gly Ala Gly Gln Gly Gly Leu Gly Ser Gln Gly Ala Gly
   340 345 350
   Gln Gly Ala Gly Ala Ala Ala Ala Ala Ala Gly Gly Ala Gly Gln Gly
   355 360 365
   Gly Tyr Gly Gly Leu Gly Ser Gln Gly Ala Gly Arg Gly Gly Gln Gly
   370 375 380
   Ala Gly Ala Ala Ala Ala Ala Ala Gly Gly Ala Gly Gln Gly Gly Tyr
   385 390 395 400
   Gly Gly Leu Gly Ser Gln Gly Ala Gly Gln Gly Gly Tyr Gly Gly Leu
   405 410 415
   Gly Ser Gln Gly Ala Gly Arg Gly Gly Leu Gly Gly Gln Gly Ala Gly
   420 425 430
   Ala Ala Ala Ala Ala Ala Ala Gly Gly Ala Gly Gln Gly Gly Leu Gly
           435                 440                 445
   Ser Gln Gly Ala Gly Gln Gly Ala Gly Ala Ala Ala Ala Ala Ala Gly
       450                 455                 460
   Gly Ala Gly Gln Gly Gly Tyr Gly Gly Leu Gly Ser Gln Gly Ala Gly
   465                 470                 475                 480
   Arg Gly Gly Gln Gly Ala Gly Ala Ala Ala Ala Ala Ala Gly Gly Ala
                   485                 490                 495
   Gly Gln Gly Gly Tyr Gly Gly Leu Gly Ser Gln Gly Ala Gly Gln Gly
               500                 505                 510
   Gly Tyr Gly Gly Leu Gly Ser Gln Gly Ala Gly Arg Gly Gly Leu Gly
           515                 520                 525
   Gly Gln Gly Ala Gly Ala Ala Ala Ala Ala Ala Ala Gly Gly Ala Gly
       530                 535                 540
   Gln Gly Gly Leu Gly Ser Gln Gly Ala Gly Gln Gly Ala Gly Ala Ala
   545                 550                 555                 560
   Ala Ala Ala Ala Gly Gly Ala Gly Gln Gly Gly Tyr Gly Gly Leu Gly
                   565                 570                 575
   Ser Gln Gly Ala Gly Arg Gly Gly Gln Gly Ala Gly Ala Ala Ala Ala
               580                 585                 590
   Ala Ala Gly Gly Ala Gly Gln Gly Gly Tyr Gly Gly Leu Gly
           595                 600                 605
(2) INFORMATIONS POUR LA SEQ ID NO: 8:
   (i) CARACTERISTIQUES DE LA SEQUENCE:
      (A) LONGUEUR: 101 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE :
      (A) ORGANISME: Nephila clavipes
   (xi) DESCRIPTION DE LA SEQUENCE: SEQ ID NO: 8:
   Gly Ala Gly Gln Gly Gly Tyr Gly Gly Leu Gly Ser Gln Gly Ala Gly
   1 5 10 15
   Arg Gly Gly Leu Gly Gly Gln Gly Ala Gly Ala Ala Ala Ala Ala Ala
   20 25 30
   Ala Gly Gly Ala Gly Gln Gly Gly Leu Gly Ser Gln Gly Ala Gly Gln
   35 40 45
   Gly Ala Gly Ala Ala Ala Ala Ala Ala Gly Gly Ala Gly Gln Gly Gly
   50 55 60
   Tyr Gly Gly Leu Gly Ser Gln Gly Ala Gly Arg Gly Gly Gln Gly Ala
   65 70 75 80
   Gly Ala Ala Ala Ala Ala Ala Gly Gly Ala Gly Gln Gly Gly Tyr Gly
   85 90 95
   Gly Leu Gly Ser Gln
   100
(2) INFORMATIONS POUR LA SEQ ID NO: 9:
   (i) CARACTERISTIQUES DE LA SEQUENCE:
      (A) LONGUEUR: 601 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 9:
   Gly Ala Gly Gln Gly Gly Tyr Gly Gly Leu Gly Ser Gln Gly Ala Gly
   1 5 10 15
   Arg Gly Gly Leu Gly Gly Gln Gly Ala Gly Ala Ala Ala Ala Ala Ala
               20                  25                  30
   Ala Gly Gly Ala Gly Gln Gly Gly Leu Gly Ser Gln Gly Ala Gly Gln
           35                  40                  45
   Gly Ala Gly Ala Ala Ala Ala Ala Ala Gly Gly Ala Gly Gln Gly Gly
       50                  55                  60
   Tyr Gly Gly Leu Gly Ser Gln Gly Ala Gly Arg Gly Gly Gln Gly Ala
   65                  70                  75                  80
   Gly Ala Ala Ala Ala Ala Ala Gly Gly Ala Gly Gln Gly Gly Tyr Gly
                   85                  90                  95
   Gly Leu Gly Ser Gln Ala Gly Gln Gly Gly Tyr Gly Gly Leu Gly Ser
               100                 105                 110
   Gln Gly Ala Gly Arg Gly Gly Leu Gly Gly Gln Gly Ala Gly Ala Ala
           115                 120                 125
   Ala Ala Ala Ala Ala Gly Gly Ala Gly Gln Gly Gly Leu Gly Ser Gln
       130                 135                 140
   Gly Ala Gly Gln Gly Ala Gly Ala Ala Ala Ala Ala Ala Gly Gly Ala
   145                 150                 155                 160
   Gly Gln Gly Gly Tyr Gly Gly Leu Gly Ser Gln Gly Ala Gly Arg Gly
                   165                 170                 175
   Gly Gln Gly Ala Gly Ala Ala Ala Ala Ala Ala Gly Gly Ala Gly Gln
               180                 185                 190
   Gly Gly Tyr Gly Gly Leu Gly Ser Gln Ala Gly Gln Gly Gly Tyr Gly
           195                 200                 205
   Gly Leu Gly Ser Gln Gly Ala Gly Arg Gly Gly Leu Gly Gly Gln Gly
       210                 215                 220
   Ala Gly Ala Ala Ala Ala Ala Ala Ala Gly Gly Ala Gly Gln Gly Gly
   225                 230                 235                 240
   Leu Gly Ser Gln Gly Ala Gly Gln Gly Ala Gly Ala Ala Ala Ala Ala
                   245                 250                 255
   Ala Gly Gly Ala Gly Gln Gly Gly Tyr Gly Gly Leu Gly Ser Gln Gly
               260                 265                 270
   Ala Gly Arg Gly Gly Gln Gly Ala Gly Ala Ala Ala Ala Ala Ala Gly
           275                 280                 285
   Gly Ala Gly Gln Gly Gly Tyr Gly Gly Leu Gly Ser Gln Ala Gly Gln
   290 295 300
   Gly Gly Tyr Gly Gly Leu Gly Ser Gln Gly Ala Gly Arg Gly Gly Leu
   305 310 315 320
   Gly Gly Gln Gly Ala Gly Ala Ala Ala Ala Ala Ala Ala Gly Gly Ala
   325 330 335
   Gly Gln Gly Gly Leu Gly Ser Gln Gly Ala Gly Gln Gly Ala Gly Ala
   340 345 350
   Ala Ala Ala Ala Ala Gly Gly Ala Gly Gln Gly Gly Tyr Gly Gly Leu
   355 360 365
   Gly Ser Gln Gly Ala Gly Arg Gly Gly Gln Gly Ala Gly Ala Ala Ala
   370 375 380
   Ala Ala Ala Gly Gly Ala Gly Gln Gly Gly Tyr Gly Gly Leu Gly Ser
   385 390 395 400
   Gln Ala Gly Gln Gly Gly Tyr Gly Gly Leu Gly Ser Gln Gly Ala Gly
   405 410 415
   Arg Gly Gly Leu Gly Gly Gln Gly Ala Gly Ala Ala Ala Ala Ala Ala
   420 425 430
   Ala Gly Gly Ala Gly Gln Gly Gly Leu Gly Ser Gln Gly Ala Gly Gln
   435 440 445
   Gly Ala Gly Ala Ala Ala Ala Ala Ala Gly Gly Ala Gly Gln Gly Gly
   450 455 460
   Tyr Gly Gly Leu Gly Ser Gln Gly Ala Gly Arg Gly Gly Gln Gly Ala
   465 470 475 480
   Gly Ala Ala Ala Ala Ala Ala Gly Gly Ala Gly Gln Gly Gly Tyr Gly
   485 490 495
   Gly Leu Gly Ser Gln Ala Gly Gln Gly Gly Tyr Gly Gly Leu Gly Ser
   500 505 510
   Gln Gly Ala Gly Arg Gly Gly Leu Gly Gly Gln Gly Ala Gly Ala Ala
   515 520 525
   Ala Ala Ala Ala Ala Gly Gly Ala Gly Gln Gly Gly Leu Gly Ser Gln
   530 535 540
   Gly Ala Gly Gln Gly Ala Gly Ala Ala Ala Ala Ala Ala Gly Gly Ala
   545 550 555 560
   Gly Gln Gly Gly Tyr Gly Gly Leu Gly Ser Gln Gly Ala Gly Arg Gly
   565 570 575
   Gly Gln Gly Ala Gly Ala Ala Ala Ala Ala Ala Gly Gly Ala Gly Gln
   580 585 590
   Gly Gly Tyr Gly Gly Leu Gly Ser Gln
   595 600
(2) INFORMATIONS POUR LA SEQ ID NO: 10:
   (i) CARACTERISTIQUES DE LA SEQUENCE:
      (A) LONGUEUR: 119 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 10:
   Gly Pro Ser Gly Pro Gly Ser Ala Ala Ala Ala Ala Ala Gly Pro Gly
   1 5 10 15
   Gln Gln Gly Pro Gly Gly Tyr Gly Pro Gly Gln Gln Gly Pro Gly Gly
   20 25 30
   Tyr Gly Pro Gly Gln Gln Gly Pro Ser Gly Pro Gly Ser Ala Ala Ala
   35 40 45
   Ala Ala Ala Ala Ala Gly Pro Gly Gly Tyr Gly Pro Gly Gln Gln Gly
   50 55 60
   Pro Gly Gly Tyr Gly Pro Gly Gln Gln Gly Pro Ser Gly Pro Gly Ser
   65 70 75 80
   Ala Ala Ala Ala Ala Ala Ala Ala Ala Gly Pro Gly Gly Tyr Gly Pro
   85 90 95
   Gly Gln Gln Gly Pro Gly Gly Tyr Gly Pro Gly Gln Gln Gly Pro Gly
   100 105 110
   Gly Tyr Gly Pro Gly Gln Gln
   115
(2) INFORMATIONS POUR LA SEQ ID NO: 11:
   (i) CARACTERISTIQUES DE LA SEQUENCE:
      (A) LONGUEUR: 714 acides aminés
      (B) TYPE: acide aminé
      (C) NOMBRE DE BRINS: non-connue
      (D) CONFIGURATION: non-connue
   (ii) TYPE DE MOLECULE: peptide
   (iii) HYPOTHETIQUE: NON
   (iv) ANTI-SENS: NON
   (v) TYPE DE FRAGMENT: interne
   (vi) ORIGINE:
      (A) ORGANISME: Nephila clavipes
   (xi) DESCRIPTION DE LA SEQUENCE: SEQ ID NO: 11:
   Gly Pro Ser Gly Pro Gly Ser Ala Ala Ala Ala Ala Ala Gly Pro Gly
   1               5                   10                  15
   Gln Gln Gly Pro Gly Gly Tyr Gly Pro Gly Gln Gln Gly Pro Gly Gly
               20                  25                  30
   Tyr Gly Pro Gly Gln Gln Gly Pro Ser Gly Pro Gly Ser Ala Ala Ala
           35                  40                  45
   Ala Ala Ala Ala Ala Gly Pro Gly Gly Tyr Gly Pro Gly Gln Gln Gly
       50                  55                  60
   Pro Gly Gly Tyr Gly Pro Gly Gln Gln Gly Pro Ser Gly Pro Gly Ser
   65                  70                  75                  80
   Ala Ala Ala Ala Ala Ala Ala Ala Ala Gly Pro Gly Gly Tyr Gly Pro
                   85                  90                  95
   Gly Gln Gln Gly Pro Gly Gly Tyr Gly Pro Gly Gln Gln Gly Pro Gly
               100                 105                 110
   Gly Tyr Gly Pro Gly Gln Gln Gly Pro Ser Gly Pro Gly Ser Ala Ala
           115                     120                     125
   Ala Ala Ala Ala Gly Pro Gly Gln Gln Gly Pro Gly Gly Tyr Gly Pro
       130                     135                     140
   Gly Gln Gln Gly Pro Gly Gly Tyr Gly Pro Gly Gln Gln Gly Pro Ser
   145                 150                     155                 160
   Gly Pro Gly Ser Ala Ala Ala Ala Ala Ala Ala Ala Gly Pro Gly Gly
                   165                     170                     175
   Tyr Gly Pro Gly Gln Gln Gly Pro Gly Gly Tyr Gly Pro Gly Gln Gln
               180                     185                     190
   Gly Pro Ser Gly Pro Gly Ser Ala Ala Ala Ala Ala Ala Ala Ala Ala
           195                     200                     205
   Gly Pro Gly Gly Tyr Gly Pro Gly Gln Gln Gly Pro Gly Gly Tyr Gly
       210                     215                     220
   Pro Gly Gln Gln Gly Pro Gly Gly Tyr Gly Pro Gly Gln Gln Gly Pro
   225                 230                     235                 240
   Ser Gly Pro Gly Ser Ala Ala Ala Ala Ala Ala Gly Pro Gly Gln Gln
                   245                     250                     255
   Gly Pro Gly Gly Tyr Gly Pro Gly Gln Gln Gly Pro Gly Gly Tyr Gly
               260                     265                     270
   Pro Gly Gln Gln Gly Pro Ser Gly Pro Gly Ser Ala Ala Ala Ala Ala
           275                     280                     285
   Ala Ala Ala Gly Pro Gly Gly Tyr Gly Pro Gly Gln Gln Gly Pro Gly
       290                     295                     300
   Gly Tyr Gly Pro Gly Gln Gln Gly Pro Ser Gly Pro Gly Ser Ala Ala
   305                 310                     315                 320
   Ala Ala Ala Ala Ala Ala Ala Gly Pro Gly Gly Tyr Gly Pro Gly Gln
                   325                     330                     335
   Gln Gly Pro Gly Gly Tyr Gly Pro Gly Gln Gln Gly Pro Gly Gly Tyr
               340                     345                     350
   Gly Pro Gly Gln Gln Gly Pro Ser Gly Pro Gly Ser Ala Ala Ala Ala
           355                     360                     365
   Ala Ala Gly Pro Gly Gln Gln Gly Pro Gly Gly Tyr Gly Pro Gly Gln
       370                     375                     380
   Gln Gly Pro Gly Gly Tyr Gly Pro Gly Gln Gln Gly Pro Ser Gly Pro
   385 390 395 400
   Gly Ser Ala Ala Ala Ala Ala Ala Ala Ala Gly Pro Gly Gly Tyr Gly
   405 410 415
   Pro Gly Gln Gln Gly Pro Gly Gly Tyr Gly Pro Gly Gln Gln Gly Pro
   420 425 430
   Ser Gly Pro Gly Ser Ala Ala Ala Ala Ala Ala Ala Ala Ala Gly Pro
   435 440 445
   Gly Gly Tyr Gly Pro Gly Gln Gln Gly Pro Gly Gly Tyr Gly Pro Gly
   450 455 460
   Gln Gln Gly Pro Gly Gly Tyr Gly Pro Gly Gln Gln Gly Pro Ser Gly
   465 470 475 480
   Pro Gly Ser Ala Ala Ala Ala Ala Ala Gly Pro Gly Gln Gln Gly Pro
   485 490 495
   Gly Gly Tyr Gly Pro Gly Gln Gln Gly Pro Gly Gly Tyr Gly Pro Gly
   500 505 510
   Gln Gln Gly Pro Ser Gly Pro Gly Ser Ala Ala Ala Ala Ala Ala Ala
   515 520 525
   Ala Gly Pro Gly Gly Tyr Gly Pro Gly Gln Gln Gly Pro Gly Gly Tyr
   530 535 540
   Gly Pro Gly Gln Gln Gly Pro Ser Gly Pro Gly Ser Ala Ala Ala Ala
   545 550 555 560
   Ala Ala Ala Ala Ala Gly Pro Gly Gly Tyr Gly Pro Gly Gln Gln Gly
   565 570 575
   Pro Gly Gly Tyr Gly Pro Gly Gln Gln Gly Pro Gly Gly Tyr Gly Pro
   580 585 590
   Gly Gln Gln Gly Pro Ser Gly Pro Gly Ser Ala Ala Ala Ala Ala Ala
   595 600 605
   Gly Pro Gly Gln Gln Gly Pro Gly Gly Tyr Gly Pro Gly Gln Gln Gly
   610 615 620
   Pro Gly Gly Tyr Gly Pro Gly Gln Gln Gly Pro Ser Gly Pro Gly Ser
   625 630 635 640
   Ala Ala Ala Ala Ala Ala Ala Ala Gly Pro Gly Gly Tyr Gly Pro Gly
   645 650 655
   Gln Gln Gly Pro Gly Gly Tyr Gly Pro Gly Gln Gln Gly Pro Ser Gly
                   660                 665                 670
   Pro Gly Ser Ala Ala Ala Ala Ala Ala Ala Ala Ala Gly Pro Gly Gly
               675                 680                 685
   Tyr Gly Pro Gly Gln Gln Gly Pro Gly Gly Tyr Gly Pro Gly Gln Gln
       690                 695                 700
   Gly Pro Gly Gly Tyr Gly Pro Gly Gln Gln
   705                 710